Europäisches Patentamt

(19) European Patent Office

Office européen des brevets

(11) Publication number: **0 318 493 B1**

# EUROPEAN PATENT SPECIFICATION

(12)

(45) Date of publication of patent specification :
**11.09.91 Bulletin 91/37**

(51) Int. Cl.⁵ : **G01M 3/16, A61L 2/26**

(21) Application number : **87905238.9**

(22) Date of filing : **12.08.87**

(86) International application number :
**PCT/GB87/00567**

(87) International publication number :
**WO 88/01372 25.02.88 Gazette 88/05**

(54) **A TEST DEVICE FOR A POROUS LOADS STERILIZER.**

(30) Priority : **13.08.86 GB 8619682**

(43) Date of publication of application :
**07.06.89 Bulletin 89/23**

(45) Publication of the grant of the patent :
**11.09.91 Bulletin 91/37**

(84) Designated Contracting States :
**AT BE CH DE FR IT LI LU NL SE**

(56) References cited :
**EP-A- 0 132 107**
**EP-A- 0 155 933**
**DE-U- 8 523 448**
**US-A- 4 486 387**

(73) Proprietor : **THE VICTORIA UNIVERSITY OF MANCHESTER**
**Oxford Road**
**Manchester. M13 9PL (GB)**

(72) Inventor : **HAMBLETON, Roger**
**5 Kings Drive**
**Marple Stockport (GB)**
Inventor : **THORNBER, Michael, Neil**
**'Woodstock', 195 Greenmount Lane**
**Heaton, Bolton BL1 5JE (GB)**

(74) Representative : **Ajello, Michael John**
**207 Moss Lane**
**Bramhall, Stockport, Cheshire SK7 1BA (GB)**

EP 0 318 493 B1

## Description

THIS INVENTION concerns a device for testing the efficiency of sterilizers such as those used in hospitals for sterilizing porous loads, and for detecting the presence of air in such sterilizers. A test device of this kind is described in British patent specification GB 2143322 and includes a plurality of porous masses of substantially man-made material with an indicator sheet sandwiched between them, and the whole assembly is held in close superimposed relationship by means which are permeable to allow the free passage of air and steam to the external surfaces of the masses.

Sterilizers for porous loads require periodic engineer's checks to be carried out with test devices using thermocouples to provide an accurate reading of temperatures within the porous masses, and the present invention is concerned with an adaptation of the test device described and claimed in GB 2143322 to enable it to be used in engineer's tests during commissioning and recommissioning of sterilizers, and during quarterly and annual maintenance checks.

Engineer's tests require the simultaneous use of up to five thermocouples to be located at different positions on the test device within the sterilizer, typically, one in the free space within the sterilizer chamber, one or more within the mass of the test device and one in the condensate drain passage of the sterilizer chamber.

According to the present invention there is provided a device for detecting the presence of air in a steam sterilizer for porous loads, comprising at least two porous masses (10) of substantially man-made material, the material of said masses (10) being selected as one which will not undergo an exothermic reaction with steam under the conditions within a sterilizer under test, and means (15, 16) for removably holding the masses in close super-imposed relationship, said means being permeable to allow air and steam to pass freely into and out of the porous masses, characterised by at least one thermocouple and conductor leads (12) housed within a flat envelope (13) open along at least one edge region to permit said leads (12) to protrude therefrom and of a material having sufficient thickness and compressibility to accommodate the protruding leads (12), said envelope (13) being sandwiched between the masses (10) thus to be in intimate contact therewith.

An embodiment of the invention will be described, by way of example only, with reference to the accompanying drawings, in which :

Fig. 1 is an exploded perspective view of a test device made in accordance with the invention ;
Fig. 2 is a similar view showing the device assembled and closed ;
Fig. 3 is a similar view of the device upturned into an operative position with a further optional device attached to carry a thermocouple in free space ;
and Fig. 4 is a view similar to Fig. 2 prior to closure of the device and showing a removable tool to serve as an aid to assembly.

Referring now to the drawings, a test device made in accordance with one embodiment of the invention comprises four porous masses 10 each formed from a stack of single rectangular sheets of a side dimension between 10 cm and 20 cm, of a man-made woven or non-woven material such as spun bonded polypropylene having a weight in the region of 50 to 150 grammes per square metre. The material is of a kind which will not undergo an exothermic reaction with steam under the conditions within a sterilizer under test. Each stack contains an appropriate number (typically about 80) of such sheets arranged in close superimposed relationship and enclosed by perforated shrink wrapping film for convenience to produce a block of some 4 cm in height. The overall height of the blocks making up the device is between 10 cm and 20 cm.

The four blocks or masses 10 are superimposed but interposed by thermocouples generally indicated at 11. Each thermocouple consists of a pair of conductor leads 12 which join at the thermocouple itself (not visible), and the latter with its conductor leads is enclosed within a flat rectangular envelope 13 open along at least one side to permit the leads 12 to protrude therefrom and consisting of a material identical or similar to the man-made material from which the masses 10 are produced. For convenience, the envelopes 13 may be produced by folding a sheet of such material and then stitching along the two sides normal to the fold line.

Alternatively, the "envelopes" may consist of two single sheets or a single larger sheet folded about its centre line to conform to the cross-sectional dimensions of the stack.

Typically, the blocks 10 and envelopes 13 are between 10 cm and 20 cm square, and the material of the envelopes 13 is sufficiently thick and compressible to accommodate the leads 12 without any noticable increased thickness along the lines occupied by the leads. The housing of the leads in this way minimises the likelihood of steam tracking along the leads towards the thermocouple, owing to the effective seal created around the leads by the fabric of the envelope.

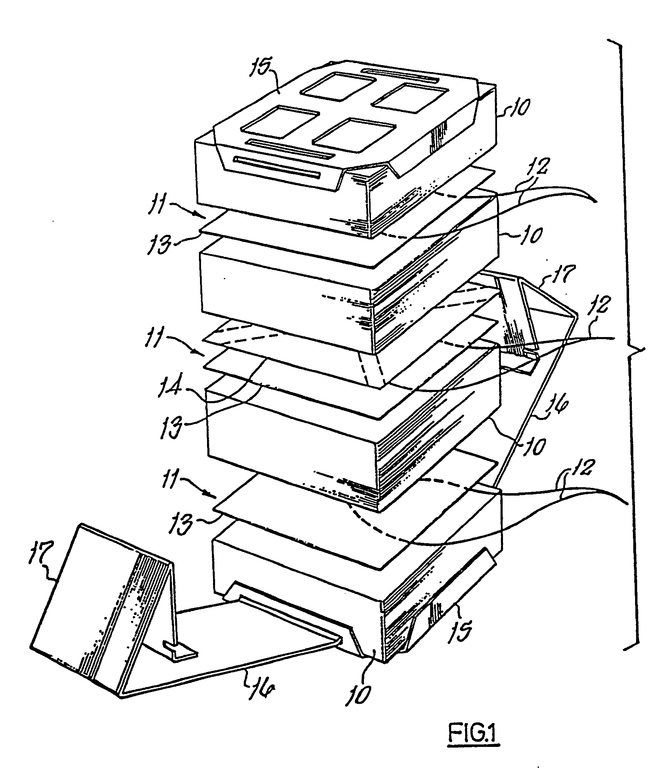

An indicator 14 consisting of a sheet of paper bearing, for example, strips of so-called autoclave tape which is adapted to undergo a visual change under moist heat sterilizing conditions, and of the kind used for carrying out a normal test for such a device, can also be included in the engineer's test along with the thermocouples, and will be disposed, as illustrated in Fig. 1, at about the mid-point of the stack of blocks 10.

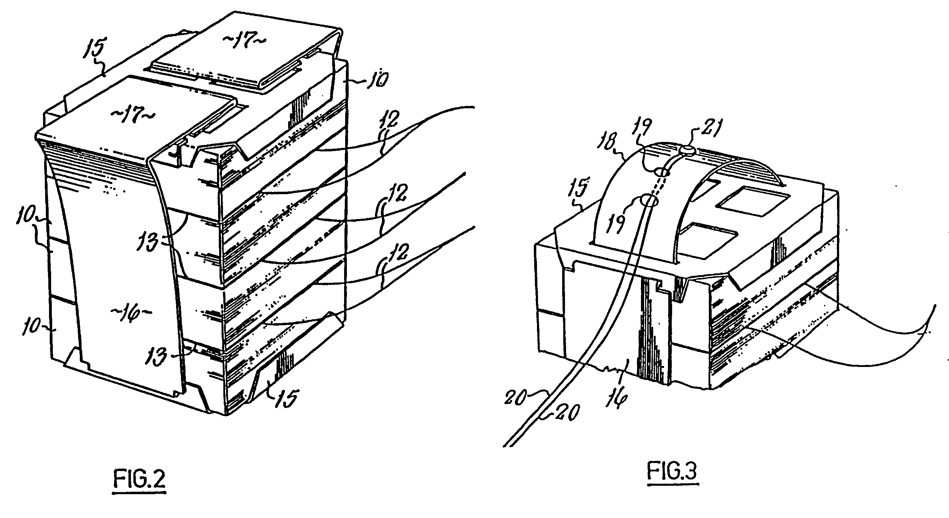

The pack is assembled and held in close superimposed relationship by means of a stainless steel clamp which consists of two square end plates 15 slotted to receive a pair of resilient side panels 16 which act as compression springs. As can be seen from Fig. 2, the side panels 16 are shaped to present a pair of broad stabilising feet 17 when the pack is assembled as in Fig. 2 and overturned to rest upon them.

Fig. 3 illustrates an optional resilient plastics bridging piece 18 which is located in appropriate slots of the upper end plate 15 and which is provided with a series of apertures 19 through which the conductor wires 20 of an additional thermocouple 21 may pass to position the latter at a height of approximately 5 cm above the pock. The piece 18 may be omitted, however, since under certain circumstances it may act as a shield to the thermocouple thus providing a disadvantageously false temperature recording.

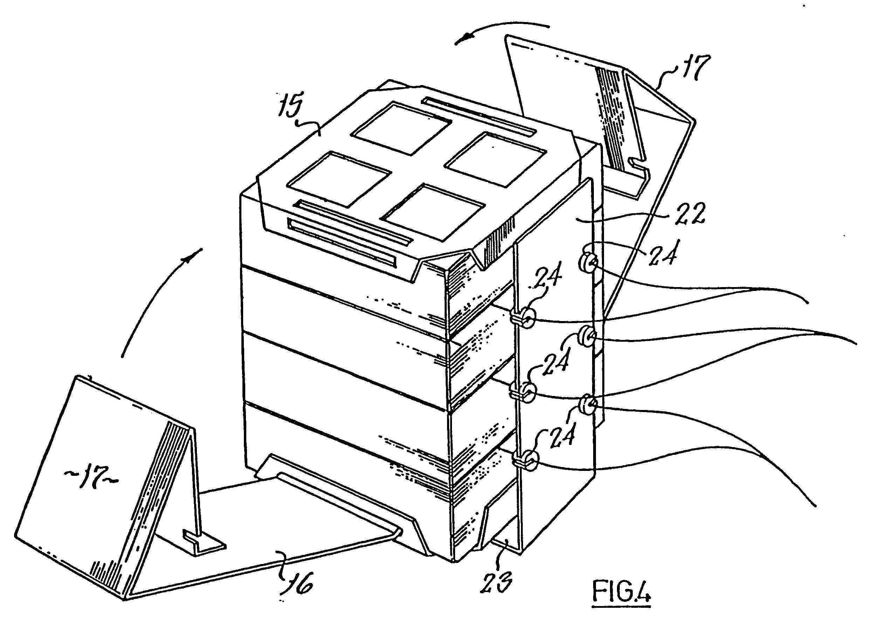

Referring now to Fig. 4, as an aid to assembly of the test device, there is provided a stainless steel stand 22 having a right angled foot 23 which is located beneath the device. Three pairs of guide members such as resilient linings 24 are held captive in slotted edge regions of the stand 22 at heights thereon equivalent to those at which the thermocouples are placed within the test device pack. With this arrangement, the delicate thermocouple leads 12 can be pre-located, and supported with vertical and level alignment in the guides 24 leaving the user's hands free to assemble the blocks or masses 10 and to place over each thermocouple in turn, an envelope 13. Once the pack is assembled, the conductor wires 12 which are thus supported by the pack, are readily removed from the linings 24 which are slotted for this purpose, and the stand 22 can be removed.

Preferably, the envelopes 13 are intended to be disposed of after each test so that any indentation or channel in their material created by the thermocouple wires at the elevated pressure and temperature experienced during the test, cannot permit steam to track inwardly along such indentation or channel during a subsequent test.

In use, the device is placed in a steam sterilizer which then undergoes a normal operational cycle or special test cycle. An additional thermocouple is placed in the chamber drain passage and, if bridging piece 18 is omitted, also in the chamber free space about 5 cm above the stack. Readings of temperature are recorded in the usual way from the thermocouples at different positions throughout the sterilizer, so that an indication is provided of the presence or absence of air within the sterilizer and of the steam penetration pattern into the device, during the test.

The invention is not limited to the above details. For example, the vertical thickness of the blocks may not be uniform, and the levels of the thermocouples within the stack can be selected according to the test requirements. In a further specific example, the blocks from top to bottom of the pack may contain respectively 20, 140, 140 and 20 sheets, and thus measure respectively, 2.5 cm, 5.5 cm, 5.5 cm and 2.5 cm. There will usually be a division at the mid-point, with each half measuring between 5 cm and 10 cm in height and consisting of one or more individual blocks.

The materials from which the clamp and stand are constructed may be other than stainless steel. Anodised aluminium or alloys containing such may be used, or polymeric materials either alone or in combination with metals.

## Claims

1. A device for testing the presence of air in a steam sterilizer for porous loads, comprising at least two porous masses (10) of substantially man-made material, the material of said masses (10) being selected as one which will not undergo an exothermic reaction with steam under the conditions within a sterilizer under test, and means (15, 16) for removably holding the masses in close super-imposed relationship, said means being permeable to allow air and steam to pass freely into and out of the porous masses, characterised by at least one thermocouple and conductor leads (12) housed within a flat envelope (13) open along at least one edge region to permit said leads (12) to protrude therefrom and of a material having sufficient thickness and compressibility to accommodate the protruding leads (12), said envelope (13) being sandwiched between the masses (10) thus to be in intimate contact therewith.

2. A test device according to Claim 1, in which said porous masses are formed from a stack of single rectangular sheets of a man-made material such as spun-bonded polypropylene, having a weight in the region of 50 to 150 grammes per square metre.

3. A test device according to any preceding claim, wherein each said mass is enclosed by perforated shrink wrapping film.

4. A test device according to any preceding claim, wherein said envelope is formed from a sheet of substantially man-made material folded, to conform to the cross-sectional dimensions of the stack and joined along two sides normal to the fold line.

5. A test device according to any one of Claims 1 to 3, wherein said envelope is formed from two superimposed single sheets of said material.

6. A test device according to any preceding claim, wherein said envelope is formed from spun-bonded polypropylene having a weight in the region of 50 to 150 grammes per square metre.

7. A test device according to any preceding claim, comprising four of said porous masses, interposed by thermocouples housed within flat envelopes, there being an indicator sheet interposed between one pair of adjacent masses.

8. A test device according to any preceding claim, wherein said holding means comprises upper and lower end plates between which the masses and thermocouples are enclosed and compressed by a pair of resilient side panels.

9. A test device according to any preceding claim, including a bridging piece located at an upper end of the device and adapted to retain a further thermocouple at a predetermined distance in free space above the device.

10. A test device according to any preceding claim, including a removable tool to serve as an aid to assembly of the device and comprising a stand having a foot to be located beneath the device and guide means adapted removably to retain the conductor leads of a thermocouple such that, after assembly, the wires may be readily removed therefrom and the tool removed from the device.

11. A test device according to Claim 10, wherein said stand is slotted to receive resilient linings serving as said guide means.

12. A test device according to any preceding claim, wherein the holding means is of stainless steel.

13. A test device according to any preceding claim, wherein said holding means is constructed, at least in part, of anodised aluminium.

14. A test device according to any preceding claim, wherein said holding means is constructed, at least in part, of a polymeric material.

15. A test device according to any preceding claim, wherein said porous masses making up the device are between 10 cm and 20 cm in height, each having a side dimension of between 10 cm and 20 cm, and the or each envelope has a side dimension of between 10 cm and 20 cm, the material of the or each envelope being sufficiently thick and compressible to accommodate the conductor leads without any noticable increased thickness along the lines occupied by the leads.

16. A test device according to any preceding claim, wherein the masses are divided at or about a mid-point in the overall height of the device, such that each part is between 9 cm and 10 cm in height and consists of one or more individual blocks.

## Patentansprüche

1. Vorrichtung zum Testen des Vorhandenseins von Luft in einem Dampfsterilisator für poröse Güter, die wenigstens zwei poröse Massen (10) aus im wesentlichen künstlichem Material, wobei als Material der Massen (10) ein Material ausgewählt wird, das mit Dampf unter den Bedingungen innerhalb eines sich im Test befindlichen Sterilisators keine exotherme Reaktion eingeht, und eine Einrichtung (15, 16) zum lösbaren Halten der Massen in enger überlagerter Beziehung aufweist, wobei die Einrichtung durchlässig ist, um Luft und Dampf zu gestatten, frei in die und aus den porösen Massen zu gelangen, gekennzeichnet durch wenigstens ein Thermoelement und Anschlußleitungen (12), die innerhalb einer flachen Hülle (13) untergebracht sind, welche längs wenigstens eines Randgebietes offen ist, damit die Leitungen (12) herausgeführt werden können, und aus einem Material besteht, welches eine ausreichende Dicke und Kompressibilität hat, um die herausgeführten Leitungen (12) aufnehmen zu können, wobei die Hülle (13) zwischen die Massen (10) eingelegt wird und so mit diesen in innigen Kontakt kommt.

2. Testvorrichtung nach Anspruch 1, bei der die porösen Massen aus einem Stapel aus einzelnen rechteckigen Bögen künstlichen Materials wie Spinnvliespolypropylen mit einem Gewicht in dem Bereich von 50 bis 150 Gramm pro Quadratmeter gebildet sind.

3. Testvorrichtung nach irgendeinem vorhergehenden Anspruch, wobei jede Masse durch perforierte Schrumpfpackfolie umschlossen ist.

4. Testvorrichtung nach irgendeinem vorhergehendem Anspruch, wobei die Hülle aus einem Bogen aus im wesentlichen künstlichem Material gebildet ist, der so gefaltet ist, daß er den Querschnittsabmessungen des Stapels angepaßt ist, und längs der beiden zu der Faltlinie normalen Seiten verbunden ist.

5. Testvorrichtung nach einem der Ansprüche 1 bis 3, wobei die Hülle aus zwei überlagerten einzelnen Bögen des Materials gebildet ist.

6. Testvorrichtung nach irgendeinem vorhergehendem Anspruch, wobei die Hülle aus Spinnvliespolypropylen mit einem Gewicht in dem Bereich von 50 bis 150 Gramm pro Quadratmeter gebildet ist.

7. Testvorrichtung nach irgendeinem vorhergehendem Anspruch, die vier poröse Massen aufweist, zwischen denen Thermoelemente angeordnet sind, welche in flachen Hüllen untergebracht sind, wobei ein Indikatorblatt zwischen einem Paar benachbarter Massen angeordnet ist.

8. Testvorrichtung nach irgendeinem vorhergehenden Anspruch, wobei die Halteeinrichtung eine obere und eine untere Endplatte aufweist, zwischen denen die Massen und die Thermoelemente eingeschlossen und durch ein Paar elastischer Seitenplatten zusammengedrückt sind.

9. Testvorrichtung nach irgendeinem vorhergehenden Anspruch, mit einem Überbrückungsstück, das an einem oberen Ende der Vorrichtung angeordnet ist und dazu dient, ein weiteres Thermoelement in einem vorbestimmten Abstand in dem freien Raum über der Vorrichtung zu halten.

10. Testvorrichtung nach irgendeinem vorhergehenden Anspruch, mit einem entfernbaren Werkzeug, das als Hilfe beim Zusammenbauen der Vorrichtung dient und ein Gestell aufweist, das einen Fuß hat, der unter der Vorrichtung angeordnet wird, und Führungseinrichtungen zum lösbaren Halten der Anschlußleitungen eines Thermoelements derart, daß nach dem Zusammenbauen die Drähte leicht daraus entnommen werden können und das Wertzeug von der Vorrichtung entfernt werden kann.

11. Testvorrichtung nach Anspruch 10, wobei das Gestell zum Aufnehmen von elantischen Futtern, die als Führungseinrichtungen dienen, geschlitzt ist.

12. Testvorrichtung nach irgendeinem vorhergehenden Anspruch, wobei die Halteeinrichtung aus rostfreiem Stahl besteht.

13. Testvorrichtung nach irgendeinem vorhergehenden Anspruch, wobei die Halteeinrichtung, wenigstens zum Teil, aus eloxiertem Aluminium aufgebaut ist.

14. Testvorrichtung nach irgendeinem vorhergehenden Anspruch, wobei die Halteeinrichtung, wenigstens zum Teil, aus einem polymeren Material aufgebaut ist.

15. Testvorrichtung nach irgendeinem vorhergehenden Anspruch, wobei die porösen Massen, aus denen die Vorrichtung aufgebaut ist, zwischen 10 cm und 20 cm hoch sind, jeweils eine Seitenabmessung zwischen 10 cm und 20 cm haben und die oder jede Hülle eine Seitenabmessung zwischen 10 cm und 20 cm hat und das Material der oder jeder Hülle ausreichend dick und kompressibel ist, um die Anschlußleitungen ohne nennenswerte vergrößerte Dicke längs der durch die Leitungen eingenommenen Linien aufnehmen zu können.

16. Testvorrichtung nach irgendeinem vorhergehenden Anspruch, wobei die Massen in oder etwa in der Mitte der Gesamthöhe der Vorrichtung unterteilt sind, so daß jeder Teil zwischen 5 cm und 10 cm hoch ist und aus einem oder mehreren einzelnen Blöcken besteht.

**Revendications**

1. Dispositif pour le contrôle de la présence d'air dans un stérilisateur à vapeur pour charges poreuses, comprenant au moins deux masses poreuses (10) d'une matière essentiellement artificielle, la matière des dites masses (10) étant choisie de façon à ne pas subir de réaction exothermique à la vapeur dans les conditions qui règnent dans un stérilisateur en cours d'épreuve, et comprenant des moyens amovibles (15, 16) destinés à maintenir les masses serrées et empilées les unes sur les autres, lesdits moyens étant perméables pour permettre le libre passage d'air et de vapeur vers les et hors des masses poreuses, caractérisé en ce qu'il comprend au moins un thermocouple et des fils conducteurs (12) englobés dans une enveloppe aplatie (13), qui est ouverte le long d'au moins un de ses bords, de façon à permettre aux fils de sortir de l'enveloppe, et qui est faite en une matière dotée d'une épaisseur suffisante et d'une compressibilité permettant d'y loger les fils sortants (12), ladite enveloppe (13) étant intercalée entre les masses (10) de manière à être en contact intime avec celles-ci.

2. Dispositif de contrôle selon la revendication 1, caractérisé en ce que lesdites masses poreuses (10) sont constituées d'un empilage de feuilles rectangulaires individuelles d'un matériau artificiel, tel que du polypropylène filé-lié, ayant un poids de l'ordre de 50 à 150 grammes par mètre carré.

3. Dispositif de contrôle selon l'une des revendications précédentes, caractérisé en ce que chacune des dites masses (10) est enfermée dans un film d'emballage perforé rétractible.

4. Dispositif de contrôle selon l'une des revendications précédentes, caractérisé en ce que ladite enveloppe (13) est formée par une feuille d'un matériau essentiellement artificiel, qui est repliée de manière à correspondre aux dimensions en coupe transversale de l'empilage, et est fermée le long de deux côtés perpendiculaires à la ligne de pliage.

5. Dispositif de contrôle selon l'une des revendications 1 à 3, caractérisé en ce que ladite enveloppe (13) est formée de deux feuilles individuelles superposées dudit matériau.

6. Dispositif de contrôle selon l'une des revendications précédentes, caractérisé en ce que ladite enveloppe (13) est réalisée en polypropylène filé-lié d'un poids de l'ordre de 50 à 150 grammes par mètre carré.

7. Dispositif de contrôle selon l'une des revendications précédentes, caractérisé en ce qu'il comporte quatre masses poroeuses (10) entre lesquelles sont placés des thermocouples (11) encastrés dans des enveloppes aplaties (13), une feuille d'indicateur (14) étant intercalée entre les masses d'une paire de masses adjacentes.

8. Dispositif de contrôle selon l'une des revendications précédentes, caractérisé en ce que ledit moyen de maintien comporte des plaques (15) d'embout supérieure et inférieure entre lesquelles les masses et thermocouples sont enfermés et comprimés au moyen d'une paire de panneaux latéraux élastiques (16).

9. Dispositif de contrôle selon l'une des revendications précédentes, caractérisé en ce qu'il comporte une pièce (18) de pontage disposée à l'extrémité supérieure du dispositif et conçue pour maintenir un thermocouple supplémentaire dans l'espace libre à une distance prédéterminée au-dessus du dispositif.

10. Dispositif de contrôle selon l'une des revendications précédentes, caractérisé en ce qu'il comprend un outillage amovible servant d'aide au montage du dispositif et comportant un support (22) pourvu d'un pied (23) destiné à être logé sous le dispositif, ainsi que des moyens (24) de guidage conçus pour maintenir, de façon réversible, les fils conducteurs d'un thermocouple, de telle sorte qu'après assemblage, les fils puissent être retirés aisément du support et que l'outillage puisse être retiré du dispositif.

11. Dispositif de contrôle selon la revendication 10, caractérisé en ce que ledit support est pourvu de fentes destinées à recevoir des garnitures élastiques qui constituent lesdits moyens (24) de guidage.

12. Dispositif de contrôle selon l'une des revendications précédentes, caractérisé en ce que ledit support (22) est en acier inoxydable.

13. Dispositif de contrôle selon l'une des revendications précédentes, caractérisé en ce que ledit support (22) est construit au moins partiellement en aluminium anodisé.

14. Dispositif de contrôle selon l'une des revendications précédentes, caractérisé en ce que ledit support (22) est construit au moins partiellement en un matériau polymérisé.

15. Dispositif de contrôle selon l'une des revendications précédentes, caractérisé en ce que lesdites masses poreuses (10), qui constituent le dispositif, ont une hauteur de 10 cm à 20 cm et ont chacune une longueur latérale de 10 cm à 20 cm, et que l'enveloppe (13), ou chaque enveloppe (13), a une longueur latérale de 10 cm à 20 cm, le matériau constituant l'enveloppe, ou chacune des enveloppes, étant suffisamment épais et compressible pour recevoir les fils conducteurs sans augmentation notable de son épaisseur le long du chemin décrit par les fils.

16. Dispositif de contrôle selon l'une des revendications précédentes, caractérisé en ce que les masses (10) sont divisées à ou approximativement à mi-hauteur du dispositif, de telle sorte que chaque partie ait une hauteur de 5 cm à 10 cm et soit composée d'un ou de plusieurs blocs individuels.

FIG.1

FIG.2

FIG.3

EP 0 318 493 B1

FIG.4